# EUROPEAN PATENT SPECIFICATION

(11) **EP 2 417 949 B1**
(45) Date of publication and mention of the grant of the patent: **06.09.2017**
(21) Application number: 10758823.8
(22) Date of filing: 31.03.2010
(51) Int. Cl.: A61F 13/15, A61F 13/472, A61F 13/49, A61F 13/53, A61F 13/42

(54) **ABSORBENT ARTICLE**
SAUGFÄHIGER ARTIKEL
ARTICLE ABSORBANT

(30) Priority: 31.03.2009 JP 2009088499
(43) Date of publication of application: 15.02.2012
(73) Proprietor: Unicharm Corporation, Ehime 799-0111 (JP)
(72) Inventor: KOMATSU, Shinpei, Kanonji-shi Kagawa 769-1602 (JP); NODA, Yuki, Kanonji-shi Kagawa 769-1602 (JP)
(74) Representative: Dolleymores
(86) International application number: PCT/JP2010/055920
(87) International publication number: WO 2010/114052

(56) References cited:
- WO-A1-96/19172
- WO-A1-2007/073263
- WO-A1-2008/054268
- JP-A- 2007 222 598
- JP-A- 2008 006 277
- JP-T- 2007 525 245
- US-A1- 2002 169 427
- US-A1- 2006 069 363
- US-A1- 2007 083 172
- US-B1- 6 770 064

## Description

### [Technical Field]

The present invention relates to an absorbent article including a topsheet, a backsheet and an absorber.

### [Background Art]

Some conventional absorbent articles such as disposable diapers include refreshing material disposed on a skin contact surface coming into contact with the skin of a wearer and giving cool feeling to the wearer (Patent Document 1, for example). The refreshing material is covered with agent for microencapsulation which dissolves in water, and is disposed on the skin contact surface coming into contact with the skin of the wearer. When the film dissolves due to urine of the wearer, the component of the refreshing agent is released, and gives stimulus to the wearer by coming into contact with the skin of the wearer. Thus, the wearer can notice when to change the diaper.

Meanwhile, there has been disclosed an absorbent article to which a compound (hereinafter, referred to as refreshing agent) giving refreshing feeling to a wearer is applied, for example, in order to lessen sweaty and sticky feeling while the wearer is wearing the absorbent article, in addition to the function of notifying the wearer of "when to change diaper" as described above (Patent Document 2, for example).

However, conventional absorbent articles having the function of giving refreshing feeling to the wearer as described above have the following problem. When the urine of the wearer is concentrated in a certain area of an absorber, the refreshing agent in that area is exhausted quickly, and refreshing effect dose not last long.

### [Prior Art Document]

### [Patent Document]

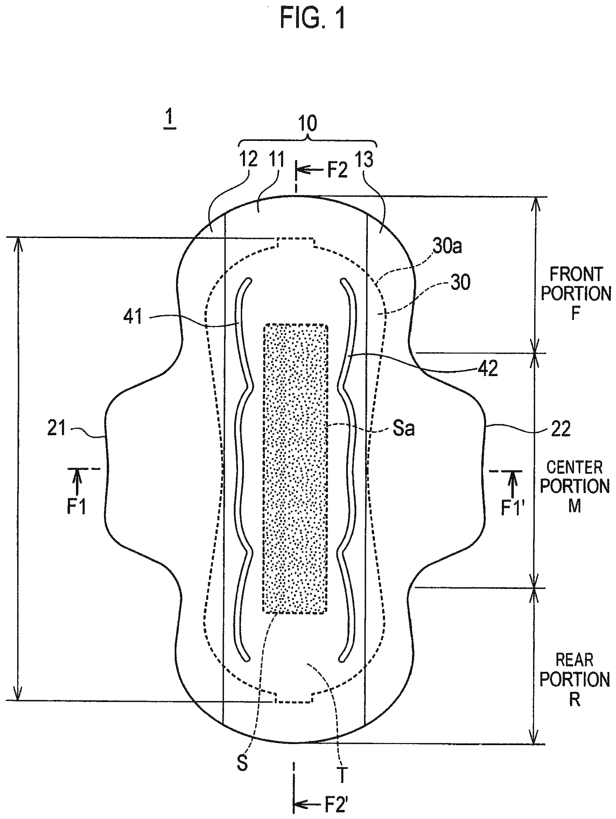

[Patent Document 1] Japanese Patent Application Publication No. 2008-006277 (page 4 and Fig. 1)
[Patent Document 2] Published Japanese Translation of PCT International Application No. 2007-525245

US 2007/0083172 A1 relates to an absorbent article including a temperature change member. The temperature change member includes temperature change material disposed with the temperature change member in a nonuniform distribution by weight across the temperature change member.

### [Summary of Invention]

An absorbent article includes: a liquid-permeable topsheet configured to allow liquid to pass through; a liquid-impermeable backsheet configured not to allow liquid to pass through; an absorber disposed between the topsheet and the backsheet; and cooling material including refreshing agent, provided between the topsheet and the backsheet. In the topsheet, an amount of the cooling material disposed per unit area is larger in a contact portion where the excretion area of a wearer comes into contact than in a portion outside the contact portion; and a coating of cooling material is applied in a pattern of stripes in an application portion.

### [Brief Description of Drawings]

[Fig. 1]
   Fig. 1 is a plan view of an absorbent article according to a reference example which does not form part of the present invention.
[Fig. 2]

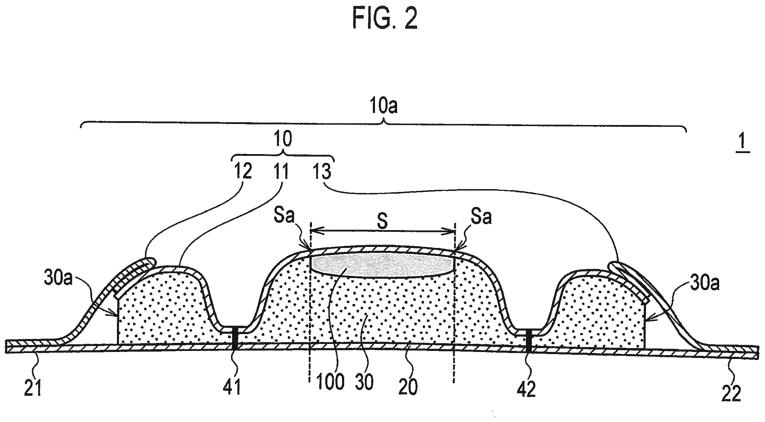

   Fig. 2 is a cross-sectional view taken along a line F1-F1' shown in Fig. 1.
[Fig. 3]

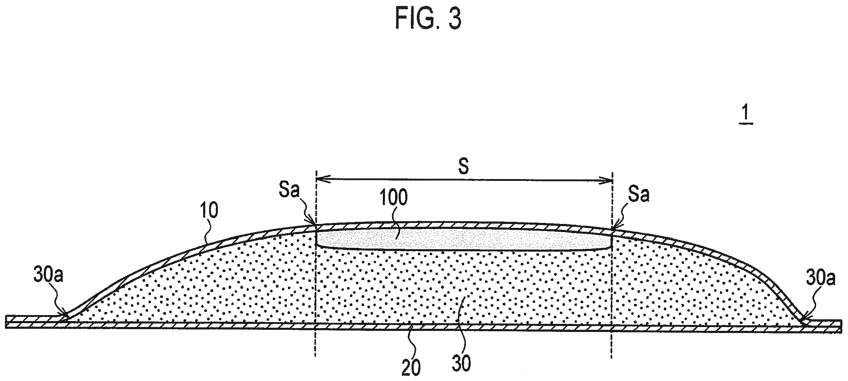

   Fig. 3 is a cross-sectional view of the absorbent article 1 taken along a line F2-F2'.
[Fig. 4]

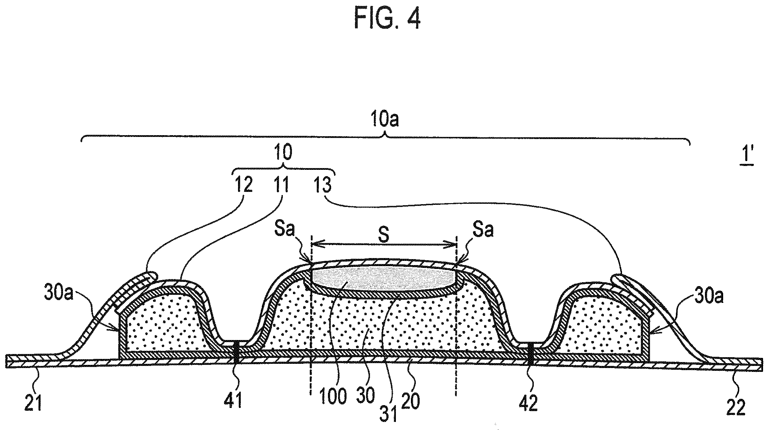

   Fig. 4 is a cross-sectional view of the absorbent article 1' taken along a line equivalent to the line F1-F1' of the absorbent article 1.
[Fig. 5]

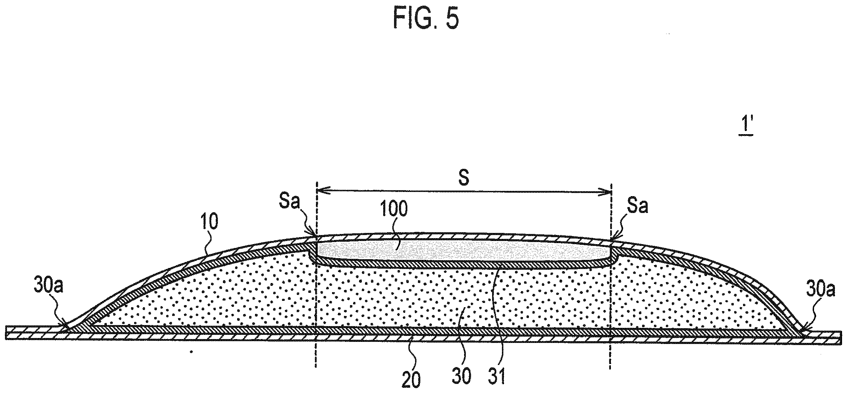

   Fig. 5 is a cross-sectional view of the absorbent article 1' taken along a line equivalent to the line F2-F2' of the absorbent article 1.
[Fig. 6]

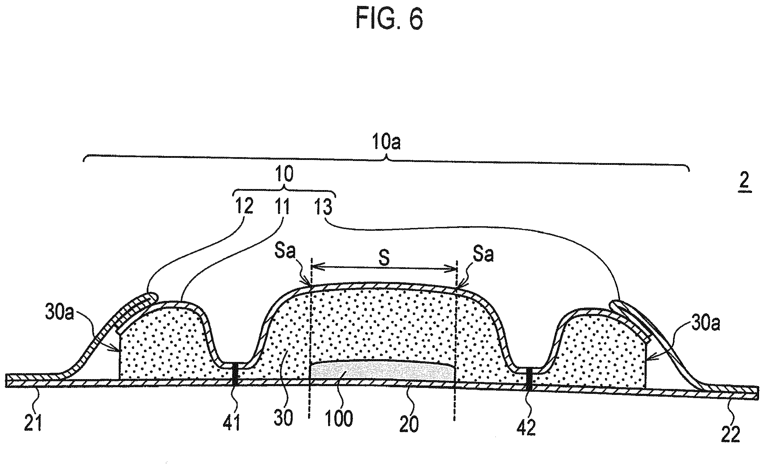

   Fig. 6 is a cross-sectional view of the absorbent article 2 taken along a line equivalent to the line F1-F1' of the absorbent article 1.
[Fig. 7]

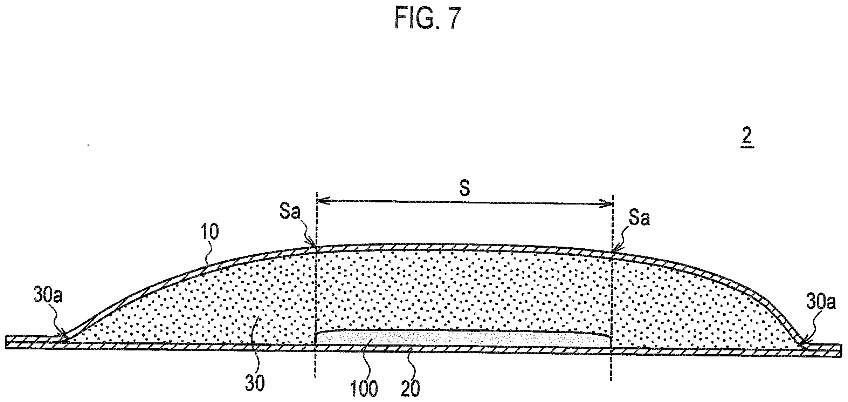

   Fig. 7 is a cross-sectional view of the absorbent article 2 taken along a line equivalent to the line F2-F2' of the absorbent article 1.
[Fig. 8]

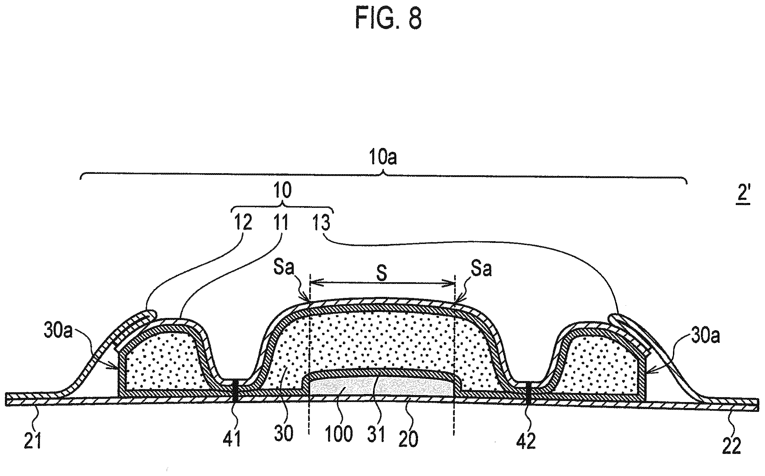

   Fig. 8 is a cross-sectional view of the absorbent article 2' taken along a line equivalent to the line F1-F1' of the absorbent article 1.
[Fig. 9]

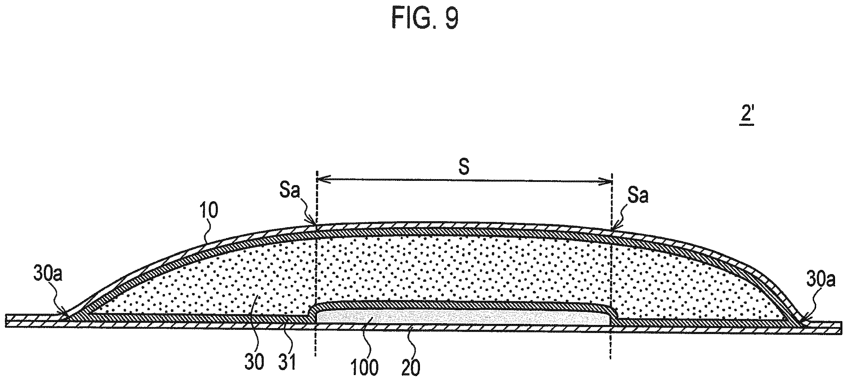

   Fig. 9 is a cross-sectional view of the absorbent article 2' taken along a line equivalent to the line F2-F2' of the absorbent article 1.
[Fig. 10]

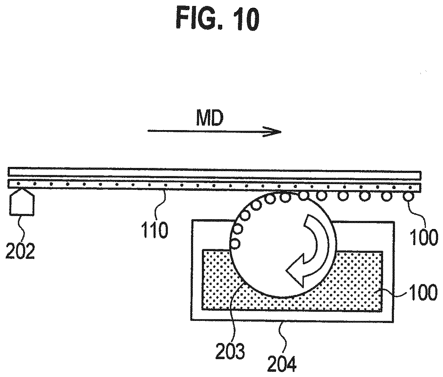

   Fig. 10 is a configuration diagram of a disposal device configured to dispose cooling material.
[Fig. 11]

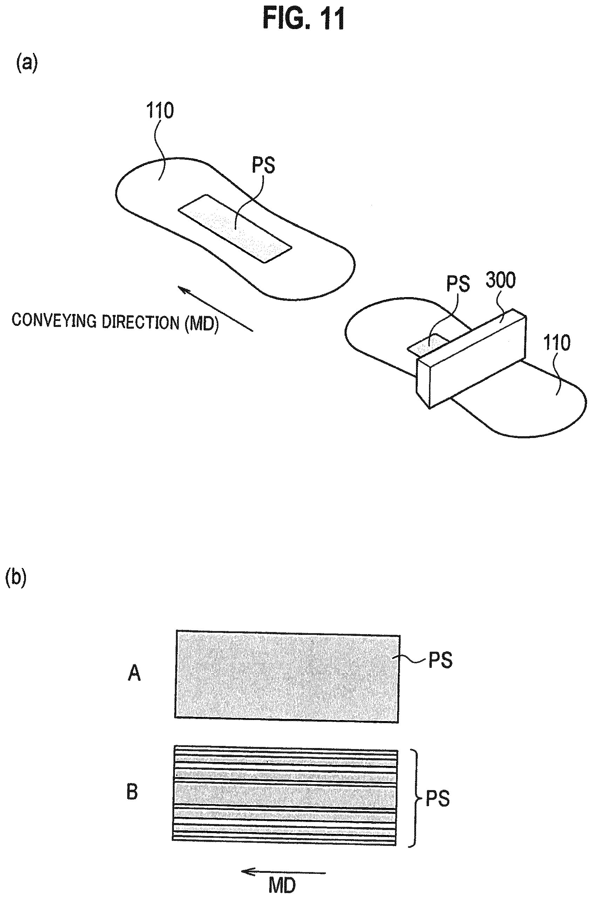

   Fig. 11(a) is a view for explaining an application method when a material 110 constituting the absorbent article is conveyed in such a direction that its longitudinal direction coincides with a conveying direction MD. Fig.11 (b) is a view for explaining examples of an application pattern of the cooling material 100.
[Fig. 12]

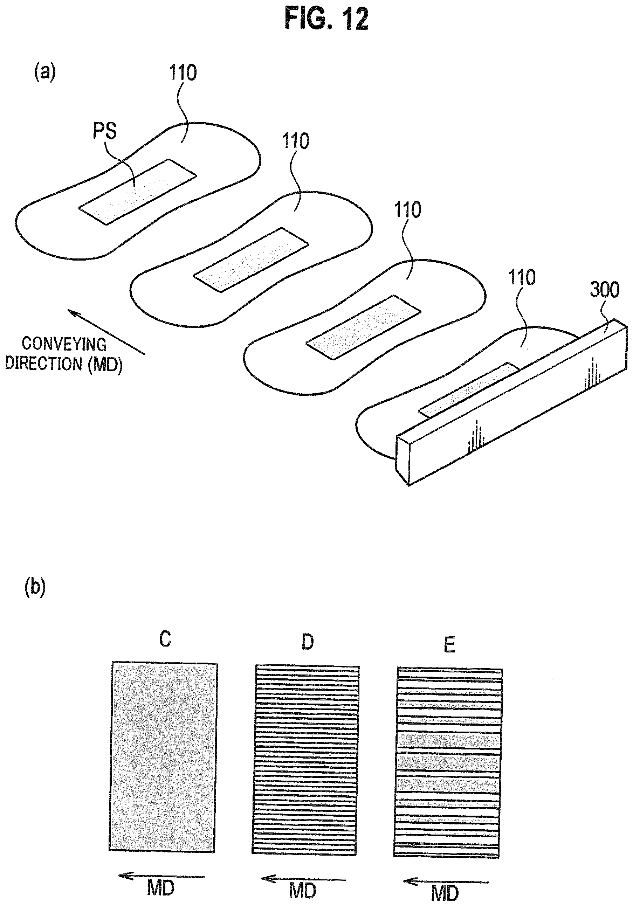

   Fig. 12 (a) is a view for explaining an application method when the material 110 constituting the absorbent article is conveyed in such a direction that its width direction coincides with the conveying direction MD. Fig. 12 (b) is a view for explaining examples of application patterns of the cooling material 100.
[Fig. 13]

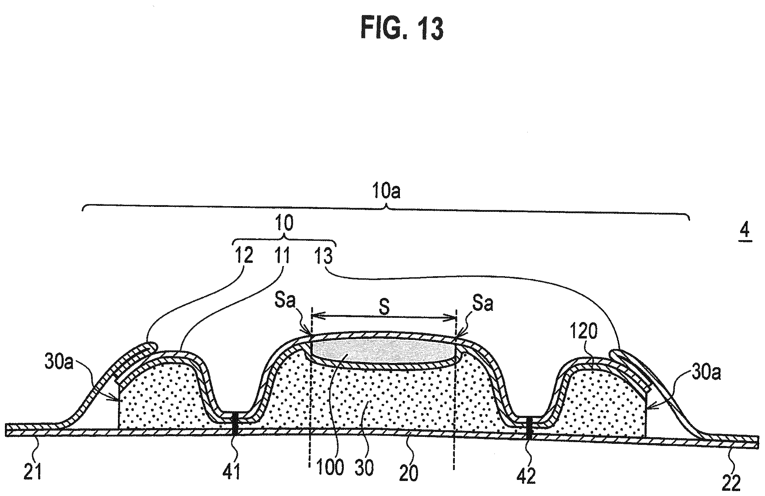

   Fig. 13 is a cross-sectional view in the width direction of an absorbent article 4 provided as another embodiment.

### [Description of Embodiments & Reference Examples]

Absorbent articles according to embodiments and to reference examples (not forming part of the invention) will be described with reference to the drawings. Note that, in the following description of the drawings, the same or similar numerals denote the same or similar portions. In addition, it should be noted that the drawings are schematic and ratios of dimensions and the like are different from actual the ones.

Therefore, specific dimensions and the like should be determined in consideration of the following description. Moreover, as a matter of course, the drawings also include portions having different dimensional relationships and ratios from each other.

### (First Reference Example)

An absorbent article according to a first reference example will be described with reference to Figs. 1 and 2. Fig. 1 is a plan view of an absorbent article 1 according to a first reference example. Fig. 2 is a cross-sectional view taken along a line F1-F1' shown in Fig. 1.

The absorbent article 1 in Fig. 1 is a sanitary napkin, for example. As shown in Fig. 1, the absorbent article 1 includes a front portion F, a center portion M, and a rear portion R. The front portion F comes into contact with the skin surface of the abdomen of a wearer. The center portion M comes into contact with the skin surface around the vagina of the wearer. The rear portion R comes into contact with the skin surface of the buttock of the wearer.

The absorbent article 1 includes a liquid-permeable topsheet 10 coming into contact with the skin of the wearer, a liquid-impermeable backsheet 20 which does not allow liquid to pass through, and an absorber 30. The absorbent article 1 includes a material 100 between the topsheet 10 and the backsheet 20. The material 100 includes a compound which gives refreshing feeling to the wearer (hereinafter, referred to as a refreshing agent). In the present reference example, the material including the refreshing agent is called cooling material 100.

The absorber 30 is disposed between the topsheet 10 and the backsheet 20. The absorber 30 is shown in a dashed line in Fig. 1. The absorber 30 is disposed in a center portion, in the longitudinal direction, of the absorbent article 1.

In the topsheet 10 of the absorbent article 1, the amount of the cooling material 100 per unit area in a contact portion S where an excretion area of the wearer comes into contact is larger than that in an area outside an edge portion Sa of the contact portion S. The cooling material 100 is disposed so that the amount of cooling material 100 per unit area becomes smaller as an area gets farther from a center portion of the absorber 30 and closer to an edge portion 30a of the absorber 30. Specifically, the cooling material 100 is disposed in the contact portion S so that the amount of cooling material 100 per unit area becomes smaller toward each of both ends in the width direction of the absorber 30. Moreover, the cooling material 100 is disposed in the contact portion S so that the amount of cooling material 100 per unit area becomes smaller toward each of both ends in the longitudinal direction of the absorber 30. A method of disposing the cooling material 100 between the topsheet 10 and the backsheet 20 in the contact portion S will be described later.

The topsheet 10 includes: a first sheet 11 which covers at least the surface, facing the wearer, of the absorber 30; and second sheets 12, 13 respectively provided on the both sides in the width direction of the first sheet 11.

The backsheet 20 includes wings 21, 22. The wings 21, 22 are formed as a pair in relative positions in the width direction of the absorbent article 1. The wings 21, 22 each extend in the width direction of the absorbent article 1 in the center portion M. The backsheet 20 has a larger width in the center portion M than in the front portion F and the rear portion R.

The first sheet 11 has approximately the same length as the backsheet 20. The end portions of the first sheet 11 each have a shape approximately the same as that of the backsheet 20. The first sheet 11 covers at least the top surface of the absorber 30.

The second sheets 12, 13 are disposed on both sides of the first sheet 11, respectively. The second sheet 12 covers the wing 21 and one side end portion of the absorber 30. One end portion extending in the longitudinal direction of the second sheet 12 forms an almost straight line, and overlaps one end portion extending in the longitudinal direction of the first sheet 11. The other end portion, extending in the longitudinal direction, of the second sheet 12 has a shape which matches the shapes of the wing 21 and a part of the periphery of the backsheet 20.

The dimension of the absorbent article 1 in the longitudinal direction is preferably in the range of 100 mm to 500 mm, more preferably in the range of 150 mm to 350 mm. Moreover, the dimension thereof in the width direction is preferably in the range of 30 mm to 200 mm, more preferably in the range of 40 mm to 180 mm.

The second sheet 13 covers the wing 22 and the other side end portion of the absorber 30. One end portion extending in the longitudinal direction of the second sheet 13 forms an almost straight line, and overlaps the other end portion extending in the longitudinal direction, of the first sheet 11. The other end portion, extending in the longitudinal direction, of the second sheet 13 has a shape which matches the shapes of the wing 22 and a part of the periphery of the backsheet 20.

Next, the position in which the cooling material 100 is disposed in the thickness direction of the absorbent article 1 of the first reference example will be described. Fig. 2 is a cross-sectional view of the absorbent article 1 taken along the line F1-F1'. Fig. 3 is a cross-sectional view of the absorbent article 1 taken along a line F2-F2'. As shown in Figs. 2 and 3, in the absorbent article 1, the cooling material 100 is disposed between the topsheet 10 and the absorber 30 in the contact portion S.

In the absorbent article 1, the first sheet 11, the second sheets 12, 13, the backsheet 20, and the absorber 30 are bonded to each other. The end portions of the first sheet 11, the second sheets 12, 13, and the backsheet 20 are bonded to each other, and are thereby sealed with the absorber 30 disposed therein.

Any one of heat embossing, ultrasonic adhesive, and hot-melt adhesive, or any combination of these may be employed as a method of bonding the topsheet 10 and the backsheet 20. The first sheet 11 and the absorber 30 are compression bonded to each other at compression bonding portions 41, 42. The compression bonding portions 41, 42 are formed respectively on the both sides in the width direction of the absorber 30, so as to extend in the longitudinal direction of the absorber 30. In the present reference example, the compression bonding portions 41, 42 are compression bonded by heat embossing.

Unillustrated adhesive is linearly applied to the surface of the backsheet 20 which comes into contact with shorts. The adhesive is applied to form multiple lines extending in the longitudinal direction of the backsheet 20. The adhesive is also applied to the surfaces of the wings 21, 22 which come into contact with the shorts. A protection sheet is attached onto each portion with the adhesive to maintain adhesiveness. The protection sheet is removed by the wearer when the absorbent article 1 is used.

Next, specific descriptions of the first sheet 11 will be provided.

The first sheet 11 is nonwoven fabric in this reference example. A material for the first sheet 11 is particularly not limited as long as the material is sheet-shaped and has a structure allowing liquid to pass through, such as woven fabric or a perforated plastic sheet. Any of natural fibers and chemical fibers may be used as a material for the woven fabric or the nonwoven fabric.

An example of the natural fibers includes cellulose such as ground pulp and cotton. Examples of the chemical fibers include: regenerated cellulose such as rayon and fibrillated rayon; semisynthetic cellulose such as acetate and triacetate; thermoplastic hydrophobic chemical fibers; thermoplastic hydrophobic chemical fibers subjected to hydrophilic processing; and the like.

Examples of the thermoplastic hydrophobic chemical fiber include: single fiber such as polyethylene (PE), polypropylene (PP), and polyethylene terephthalate (PET); fiber formed by graft polymerization of polyethylene on polypropylene; and composite fiber such as core-sheath fiber.

A method of forming a web of the nonwoven fabric may be any one of dry-type (card method, spun bond method, melt blown method, air-laid method, and the like) and wet-type methods. Any multiple methods among the dry-type and wet-type methods may be combined. Moreover, the web forming method may be a thermal bonding method, a needle punch method, a chemical bonding method, or the like. The method for forming the nonwoven fabric is not limited to the methods mentioned above.

Spunlace formed into a sheet by hydroentanglement may be used as the first sheet 11. In addition, the first sheet 11 may be nonwoven fabric having an upper layer with convexes and concaves or nonwoven fabric with convexes and concaves formed by blowing air on the nonwoven fabric in the web formation so that the nonwoven fabric has an uneven distribution of mass per unit area. Forming the first sheet 11 so that the first sheet 11 has a surface with convexes and concaves allows body fluid to be less dispersed along the surface of the first sheet 11 before the body fluid passes through the first sheet 11.

A material to be used for the second sheets 12, 13 may be chosen from the materials which may be used for the first sheet 11. However, it is preferable for the second sheets 12, 13 to have hydrophobic properties or water repellency in order to prevent menstrual blood from flowing outside the absorbent article 1 beyond the second sheets 12, 13. Specifically, spun bond nonwoven fabric, SMS nonwoven fabric or the like may be used. The second sheets 12, 13 form a surface 10a which comes into contact with the skin. Accordingly, air-through nonwoven fabric is preferably used to lessen the irritation due to friction applied to the skin.

Next, specific descriptions of the backsheet 20 will be provided.

In this reference example, the backsheet 20 may be formed of a film mainly made of polyethylene, polypropylene, or the like; an air-permeable resin film, spun bond, or a sheet formed by bonding an air-permeable resin film to nonwoven fabric such as spunlace. The backsheet 20 is preferably made of a material having enough flexibility to prevent the wear from feeling discomfort while wearing the absorbent article 1. For example, the backsheet 20 is preferably formed of a film mainly made of low density polyethylene (LDPE) resin and having a mass (g) per unit area within the range of 15 g/m² to 30 g/m².

Next, specific descriptions of the absorber 30 will be provided.

The absorber 30 includes hydrophilic fibers and pulp. Examples of the hydrophilic fibers include: cellulose such as ground pulp and cotton; regenerated cellulose such as rayon and fibrillated rayon; semisynthetic cellulose such as acetate and triacetate; particulate polymer; fibrous polymer; thermoplastic hydrophobic chemical fibers; thermoplastic hydrophobic chemical fibers subjected to hydrophilic processing; and the like. The above materials may be used singly or may be mixed together. Among the materials, the ground pulp is preferably used from the view point of low cost and easy formation of the absorber.

Hydrophilic fiber mixed with high polymer absorbent may be used as the absorber 30. In this reference example, the high polymer absorbent is particulate polymer of sodium acrylate copolymer which has absorbent properties and moisture absorption properties, or the like. Moreover, the absorber 30 may be added with particulate deodorant of silver, copper, zinc, silica, activated carbon, aluminosilicate compound, zeolite, and the like, or with particulate antibacterial agent. Furthermore, the absorber 30 may be added with particulate coolant exerting a cooling effect by endothermal reaction.

The absorber 30 may be an air-laid sheet formed by utilizing an air-laid method in which hydrophilic fibers or powder is formed into a sheet. When an air-laid sheet is used as the absorber 30, the thickness of the air-laid sheet is preferably 0.3 mm to 5.0 mm. An example of the air-laid sheet includes a sheet made of fibers and particulate polymer using binder or the like. Note that, particulate polymer in the air-laid sheet may be dispersed in layers or may exist unevenly in the thickness direction.

The absorber 30 may be embossed to prevent deformation and twisting of the absorber 30 while the wearer is using the absorbent article 1, or to adjust the thickness of the absorber 30. The absorber 30 is embossed by sending the absorber between a flat roll and an emboss roll provided with a pattern. The pattern of the emboss roll includes a grid, dots, waves, or the like. The pattern with a grid is preferable since the thickness of the absorber can be adjusted easily.

Next, specific descriptions of the cooling material 100 will be provided.

In the present reference example, it is preferable that the cooling material 100 be soluble and has a structure containing a refreshing agent. Specifically, the refreshing agent is preferably contained inside polymer cells such as clathrate compound, microcapsules, or microspheres. The refreshing agent is, for example, cyclohexanol derivative such as menthol, camphor, or thiol. When the cooling material 100 comes into contact with liquid, the polymer cells covering the refreshing agent break and the refreshing agent contained in each polymer cell is released.

The mass per unit area of the cooling material 100 is 0.1 g/m² to 50 g/m², which differs depending on the type of the refreshing agent, the type of the polymer cells, and the like. Specifically, the mass per unit area is preferably 0.5 g/m² to 5 g/m² when microspheres (manufactured by Symrise AG) containing L-menthol with a ratio of menthol being 25% is used.

As described above, in the absorbent article 1 according to the present reference example, the amount of the cooling material 100 per unit area between the topsheet 10 and the absorber 30 in the contact portion S is larger than that in a portion outside the contact portion S. Moreover, the cooling material is disposed so that the amount of the cooling material 100 per unit area becomes smaller as an area gets farther from the center portion of the contact portion S and closer to the edge portion 30a of the absorber 30. Accordingly, in the absorbent article 1, a large amount of cooling material 100 is disposed in a portion where moisture is collected. Thus, a refreshing effect on the wearer can be made to last longer.

The cooling material 100 is disposed between the topsheet 10 and the absorber 30. Thus, the cooling material 100 does not come into direct contact with the skin of the wearer. Accordingly, excessive stimulus to the wearer can be prevented. In addition, discomfort such as sweaty and sticky feelings is more surely prevented.

The cooling material 100 dissolves when coming into contact with moisture, thereby releasing the refreshing agent. The released refreshing agent comes into contact with cold sensitive receptors TRPM8 (CMR1) in the skin, and increases the threshold of the receptors by 0C° to 5C°. As a result, the sensed temperature of the skin is decreased by 0C° to 5C° while the environmental temperature of the skin remains the same. Accordingly, the sensed temperature of the wearer is decreased in an environment where the wearer feels sweaty, and thus the wearer can feel a refreshing feeling.

When the cooling material 100 with the refreshing agent contained in the microspheres is used, the microspheres may be formed to have different particle diameters so that times required for the microspheres to dissolve differ from each other depending on the diameter. As a result, the total refreshing feeling is made to last long. In a case of microcapsules, a similar effect can be obtained by forming the microcapsules to have different film thicknesses.

### (Modified Example of First Reference example)

An absorbent article 1' is a modified example of the first reference example. In the absorbent article 1', the position where cooling material 100 is disposed is different from that of the absorbent article 1 in the thickness direction. Here, since the absorbent article 1' has no external difference from the absorbent article 1, a plan view of the absorbent article 1' is omitted. Fig. 4 is a cross-sectional view of the absorbent article 1' taken along a line equivalent to the line F1-F1' of the absorbent article 1. Fig. 5 is a cross-sectional view of the absorbent article 1' taken along a line equivalent to the line F2-F2' of the absorbent article 1. As shown in Figs. 4 and 5, an absorber 30 of the absorbent article 1' is formed by covering hydrophilic fibers, high polymer absorbent, or the like with a cover material 31. In the absorbent article 1', the amount of the cooling material 100 per unit area between a topsheet 10 and the cover material 31 in a portion corresponding to the contact portion S in Fig. 1 is larger than that in a portion outside the contact portion S.

### (Second Reference example)

A description will be given of an absorbent article 2 provided as a second reference example. In the absorbent article 2, the position where cooling material 100 is disposed is different from that of the absorbent article 1 in the thickness direction. Here, since is the absorbent article 2 has no external difference from the absorbent article 1, a plan view of the absorbent article 2 is omitted. Fig. 6 is a cross-sectional view of the absorbent article 2 taken along a line equivalent to the line F1-F1' of the absorbent article 1. Fig. 7 is a cross-sectional view of the absorbent article 2 taken along a line equivalent to the line F2-F2' of the absorbent article 1. As shown in Figs. 6 and 7, in the absorbent article 2, the amount of the cooling material 100 per unit area between an absorber 30 and a backsheet 20 in a portion corresponding to the contact portion S in Fig. 1 is larger than that in a portion outside the contact portion S.

As described above, a large amount of the cooling material 100 is disposed in a portion where moisture is collected. Thus, a refreshing effect on the wearer can be last longer. Moreover, the cooling material 100 is disposed between the backsheet 20 and the absorber 30. Thus, the cooling material 100 does not come into direct contact with the skin of the wearer. Accordingly, excessive stimulus to the wearer can be prevented. In addition, discomfort such as sweaty and sticky feelings is more surely prevented.

### (Modified Example of Second reference example)

An absorbent article 2' is a modified example of the second reference example. In the absorbent article 2', the position where cooling material 100 is disposed is different from that of the absorbent article 1 in the thickness direction. Here, since the absorbent article 2' has no external difference from the absorbent article 1, a plan view of the absorbent article 2' is omitted. Fig. 8 is a cross-sectional view of the absorbent article 2' taken along a line equivalent to the line F1-F1' of the absorbent article 1. Fig. 9 is a cross-sectional view of the absorbent article 2' taken along a line equivalent to the line F2-F2' of the absorbent article 1. As shown in Figs. 8 and 9, an absorber 30 of the absorbent article 2' is formed by covering hydrophilic fibers, high polymer absorbent, or the like with a cover material 31. In the absorbent article 2', the amount of the cooling material 100 per unit area between the cover material 31 and a backsheet 20 in a portion corresponding to the contact portion S in Fig. 1 is larger than that in a portion outside the contact portion S.

### (First Method for Disposing Cooling material)

The first method is given as a method for disposing the cooling material 100 in the respective positions illustrated in the above reference examples and does not form part of the present invention. A first disposing method is a method for disposing the cooling material 100 in which refreshing agent is contained inside polymer cells. Specifically, the cooling material 100 is attached to a portion applied with hot melt adhesive by using a roll.

Fig. 10 is a configuration diagram for explaining the outline of a disposal device 200 configured to dispose the cooling material 100. As shown in Fig. 10, the disposal device 200 includes: a conveyance part 201 for conveying a material 110 in a given direction (MD direction); and an HMA coater 202 for applying hot melt adhesive (hereinafter, referred to as HMA) onto a surface of the material 110. The disposal device 200 additionally includes: a surface processing roll 203 for disposing the cooling material 100 onto the surface of the material 110 to which the HMA is applied; and a storage tank 204 in which the cooling material 100 is stored. Fine holes are formed in a surface of the surface processing roll 203.

The fine holes formed in the surface of the surface processing roll 203 are filled with the cooling material 100 when the surface processing roll 203 is rotated while being in contact with the cooling material 100 stored in the storage tank 204. When the surface processing roll 203 comes into contact with the surface of the material 110, the cooling material 100 is transferred onto the surface of the material 110 to which the HMA is applied.

The cooling material 100 is partially disposed at positions corresponding to the contact portion S of the absorber 30 by partially forming the fine holes in the surface of the surface processing roll 203. The fine holes are formed only in positions, corresponding to the contact portion S of the absorber 30, of the surface processing roll 203. Moreover, the HMA only needs to be applied to the positions, corresponding to the contact portion S, of the surface of the material 110.

The material 110 is the absorber 30, for example. The disposal device 200 in Fig. 10 disposes the cooling material 100 onto the back surface of the topsheet 10 (surface opposite to a skin contact surface coming into contact with the skin of the wearer) or onto a side, being closer to the skin, of the absorber 30. The cooling material 100 is bonded to the surface of the material 110 by the HMA. This allows the amount of the cooling material 100 disposed per unit area on the material 110 to be changed by changing the application interval and the application area of the HMA. The application amount of the HMA is preferably 5 g/m² to 100 g/m². Moreover, the application interval of the HMA in the width direction with respect to the MD direction is 0.5 mm, and the application interval in the MD direction is 0.5 mm to 10 mm, more preferably between 0.5 mm and 2 mm both inclusive. The coating method of the HMA is spiral coating, for example. The spiral coating is preferably used since the method does not inhibit penetrating properties of liquid.

A method of disposing a different sheet (referred to as a secondary sheet) with the cooling material 100 disposed thereon to a certain position may be employed instead of the method of directly disposing the cooling material 100 onto the back surface of the topsheet 10 or onto the side, being closer to the skin, of the absorber 30. The certain position is a position between the topsheet 10 and the absorber 30, for example.

In a case of using material having low resistance to heat for the backsheet 20, application of the HMA onto the backsheet 20 may be difficult. In this case, the cooling material 100 is preferably disposed on the back surfaces of the second sheets 12, 13.

The disposing method shown in Fig. 10 which uses the disposal device 200 is not limited to the case of disposing the cooling material 100 in which the refreshing agent is contained inside the polymer cells. The method is also effective when the cooling material 100 has a particle shape. For example, the disposing method can be employed in a case of disposing the cooling material 100 in which the refreshing agent is held by a porous material.

### (Second Method for Disposing Cooling material)

The second method is given as a method for disposing the cooling material 100 in the respective positions illustrated in the following embodiments of the invention. The second disposing method is a method of applying refreshing agent in a form of solution onto certain positions, the refreshing agent being one soluble to specific solvent, such as lactic acid menthyl or menthone glycerin acetal. Coating using a coater can be employed for the application of the refreshing agent in a form of solution. Figs. 11 (a) and 11 (b) are views for explaining the outline of a coater device.

A coater 300 applies the cooling material 100 onto a portion corresponding to the contact portion S. The cooling material 100 is applied onto an application portion PS. Here, two cases can be assumed: one case where a material 110 constituting the absorbent article is conveyed in such a direction that its longitudinal direction coincides with a conveying direction MD (Fig. 11 (a)), and the other case where the material 110 constituting the absorbent article is conveyed in such a direction that its width direction coincides with the conveying direction MD (Fig. 12 (a)).

Fig. 11 (b) shows examples of an application pattern which can be used to dispose the cooling material 100 in the application portion PS when the material 110 constituting the absorbent article is conveyed in such a direction that its longitudinal direction coincides with the conveying direction MD. The examples include, for example, a pattern A (not forming part of the present invention) in which the cooling material 100 is applied to the entire surface of the application portion PS, and a pattern B in which the cooling material 100 is applied in stripes in the application portion PS. In the pattern B, the amount of the cooling material 100 disposed per unit area becomes smaller as an area gets closer to each of both ends of the contact portion S in the width direction of the absorber.

Fig .12 (b) shows examples of an application pattern which can be used to dispose the cooling material 100 in the application portion PS when the material 110 constituting the absorbent article is conveyed in such a direction that its width direction coincides with the conveying direction MD. The examples include, for example, a pattern C (not forming part of the present invention) in which the cooling material 100 is applied to the entire surface of the application portion PS, and patterns D, E in each of which the cooling material 100 is applied in stripes in the application portion PS. In the pattern E, the amount of the cooling material 100 disposed per unit area becomes smaller as an area gets closer to each of both ends of the contact portion S in the longitudinal direction of the absorber.

In the present embodiments, the amount of the cooling material 100 per unit area in the contact portion S only needs to be larger than that in the portion outside the contact portion S. In other words, the cooling material 100 may be disposed outside the contact portion S. Moreover, so-called flexo coating, gravure coating, or the like can be employed to dispose the cooling material 100 on the material 110 constituting the absorbent article as illustrated in Figs. 11 (b), or 12 (b). In such coating, the amount of the cooling material 100 per unit area can be changed by repeating the coating multiple times.

The contents of the present invention have been disclosed on the basis of the above embodiments. However, it should not be understood that the statements and the drawings which form part of this disclosure limit the embodiments. From this disclosure, various alternative embodiments, examples, and applications are apparent to those skilled in the art.

For example, the embodiments can be changed as follows. In the above embodiments, the absorbent article is described as a sanitary napkin. However, the embodiments can be applied to liners, incontinence products (referred to as incontinence pads), and the like.

In addition, the planar shape of the absorbent article is not limited to the above-described one shown in Fig. 1. The shape can be any as long as the shape fits the crotch shape of the wearer and the shape of shorts. The planar shape of the absorbent article may be any of rectangle, oval, gourd-shaped, and the like.

The absorbent article may be provided with gathers using elastic materials on both ends in the width direction of the absorber, to prevent side leakage of body fluid such as menstrual blood.

Generally, particulate polymer of sodium acrylate copolymer or the like which has absorbent properties and moisture absorption properties may be used as the high polymer absorbent. Particulate deodorant of silver, copper, zinc, silica, activated carbon, aluminosilicate compound, zeolite, or the like may be disposed between the topsheet and the backsheet in addition to the absorber, high polymer absorbent, and moisture absorber.

Some of silver, copper, zinc, silica, activated carbon, aluminosilicate compound, zeolite, electrolyte usable as the moisture absorbent, and the like have an effect of suppressing the propagation of bacteria (antibacterial effect or bactericidal effect). For example, when electrolyte having an effect of suppressing the propagation of bacteria is used as the moisture absorber, the absorbent article has an effect of suppressing the propagation of bacteria.

The absorbent article is only required to have a portion where the amount of the cooling material 100 per unit area is larger, in a position which is between the topsheet 10 and the backsheet 20 and corresponds to the contact portion S shown in Fig 1, and the position in which the cooling material 100 is disposed in the thickness direction is not limited to the above described embodiments. In the above embodiments, described is the case where the absorbent article has the topsheet 10 and the backsheet 20, and the absorber 30 is disposed between the topsheet 10 and the backsheet 20. Moreover, it is described that the absorber 30 may be covered with the cover material 31. However, another sheet (for example, the secondary sheet 120) may be further provided between the topsheet 10 and the absorber 30 (see Fig. 13). In this case, the cooling material 100 can be disposed between the topsheet 10 and the secondary sheet 120 by using the second disposing method. Note that, the cooling material 100 can be disposed between the secondary sheet 120 and the absorber 30.

As described above, the present invention naturally includes various embodiments which are not described herein. Accordingly, the technical scope of the present invention should be only determined according to the subject matter recited in the scope of claims which is appropriate based on the foregoing description.

### [Industrial Applicability]

According to the present invention, it is possible to provide an absorbent article which can improve a prolongableness of the refreshing agent.

## Claims

1. An absorbent article (1) comprising:
a liquid-permeable topsheet (10) configured to allow liquid to pass through;
a liquid-impermeable backsheet (20) configured not to allow liquid to pass through;
an absorber (30) disposed between the topsheet and the backsheet; and
cooling material (100) including refreshing agent, provided between the topsheet and the backsheet,
in the topsheet, an amount of the cooling material disposed per unit area is larger in a contact portion (S) where the excretion area of a wearer comes into contact than in a portion outside the contact portion;
a coating of the cooling material is applied in a pattern of stripes in an application portion (PS) the stripes are orthogonally oriented with respect to the longitudinal direction of the absorbent article; and
the amount of the cooling material per unit area becomes smaller as an area gets closer to each of both ends in the longitudinal direction of the absorber.

2. The absorbent article according to claim 1, wherein the cooling material is disposed between the topsheet and the absorber.

3. The absorbent article according to claim 1 or claim 2, wherein the amount of the absorber in the contact portion is larger than the amount of the absorber in a portion outside the contact portion.

4. The absorbent article according to any preceding claim, wherein the cooling material has a structure containing the refreshing agent inside the cooling material, and is soluble in water.

## Patentansprüche

1. Absorbierender Artikel (1), der Folgendes umfasst:
eine flüssigkeitsdurchlässige obere Lage (10), die konfiguriert ist, um das Durchlaufen von Flüssigkeit zu ermöglichen;
eine flüssigkeitsundurchlässige hintere Lage (20), die konfiguriert ist, um das Durchlaufen von Flüssigkeit nicht zu ermöglichen;
einen Absorber (30), der zwischen der oberen Lage und der hinteren Lage angeordnet ist; und
kühlendes Material (100), das ein Erfrischungsmittel einschließt, das zwischen der oberen Lage und der hinteren Lage bereitgestellt ist,
wobei in der oberen Lage eine Menge des kühlenden Materials, das pro Flächeneinheit angeordnet ist, größer in einem Kontaktteil (S) ist, wo die Ausscheidungsfläche eines Trägers in Kontakt kommt, als in einem Teil außerhalb des Kontaktteils;
eine Beschichtung des kühlenden Materials in einem Muster von Streifen in einem Anwendungsteil (PS) angewendet ist, die Streifen orthogonal in Bezug auf die Längsrichtung des absorbierenden Artikels ausgerichtet sind; und
die Menge des kühlenden Materials pro Flächeneinheit kleiner wird, wenn sich eine Fläche jedem der beiden Enden in der Längsrichtung des Absorbers nähert.

2. Absorbierender Artikel nach Anspruch 1, wobei das kühlende Material zwischen der oberen Lage und dem Absorber angeordnet ist.

3. Absorbierender Artikel nach Anspruch 1 oder Anspruch 2, wobei die Menge des Absorbers in dem Kontaktteil größer ist als die Menge des Absorbers in einem Teil außerhalb des Kontaktteils.

4. Absorbierender Artikel nach einem vorstehenden Anspruch, wobei das kühlende Material eine Struktur aufweist, die das Erfrischungsmittel innerhalb des kühlenden Materials enthält, und in Wasser löslich ist.

## Revendications

1. Article absorbant (1) comportant :
une feuille de dessus perméable au liquide (10) configurée pour permettre au liquide de passer au travers ;
une feuille de support imperméable au liquide (20) configurée pour ne pas laisser le liquide passer au travers ;
un élément absorbant (30) disposé entre la feuille de dessus et la feuille de support ; et
un matériau de refroidissement (100) comprenant un agent rafraîchissant, mis en oeuvre entre la feuille de dessus et la feuille de support,
dans la feuille de dessus, une quantité du matériau de refroidissement disposé par unité de surface est supérieure dans une partie de contact (S) avec laquelle la zone d'excrétion d'un utilisateur entre en contact par rapport à une partie se trouvant à l'extérieur de la partie de contact ;
un revêtement du matériau de refroidissement est appliqué en un motif de rayures dans une partie d'application (PS), les rayures sont orientées de manière orthogonale par rapport à la direction allant dans le sens longitudinal de l'article absorbant ; et
la quantité du matériau de refroidissement par unité de surface devient plus petite quand une surface se rapproche de chacune des deux extrémités dans la direction allant dans le sens longitudinal de l'élément absorbant.

2. Article absorbant selon la revendication 1, dans lequel le matériau de refroidissement est disposé entre la feuille de dessus et la feuille de support.

3. Article absorbant selon la revendication 1 ou la revendication 2, dans lequel la quantité de l'élément absorbant dans la partie de contact est supérieure à la quantité de l'élément absorbant dans une partie à l'extérieur de la partie de contact.

4. Article absorbant selon l'une quelconque des revendications précédentes, dans lequel le matériau de refroidissement a une structure contenant l'agent rafraîchissant à l'intérieur du matériau de refroidissement, et est soluble dans l'eau.
